# EUROPEAN PATENT SPECIFICATION

(11) **EP 3 250 160 B1**
(45) Date of publication and mention of the grant of the patent: **27.11.2019**
(21) Application number: 16744144.3
(22) Date of filing: 28.01.2016
(51) Int. Cl.: A61F 5/055, A61F 5/058

(54) **ADJUSTABLE COLLAR**
EINSTELLBARE HALSKRAUSE
COLLIER AJUSTABLE

(30) Priority: 28.01.2015 US 201514607840
(43) Date of publication of application: 06.12.2017
(73) Proprietor: Aspen Medical Partners, LLC, Irvine, California 92618 (US)
(72) Inventor: GARTH, Geoffrey, Irvine, California 92618 (US); BURKE, Steven, Irvine, California 92618 (US)
(74) Representative: Hutter, Anton
(86) International application number: PCT/US2016/015474
(87) International publication number: WO 2016/123408

(56) References cited:
- WO-A1-2013/059817
- US-A- 4 993 409
- US-A- 5 403 266
- US-A1- 2011 172 579
- US-A1- 2012 291 189
- US-A1- 2013 110 019
- US-A1- 2014 130 261
- US-A1- 2014 330 184

## Description

### Field of the Invention

The field of the invention is pneumatic orthosis.

### Background

The background description includes information that may be useful in understanding the present invention. It is not an admission that any of the information provided herein is prior art or relevant to the presently claimed invention, or that any publication specifically or implicitly referenced is prior art.

Efforts have been made to develop alternatives to traditional orthotic devices, which tend to be uncomfortable and suitable only for specific persons or have limited uses. For example, U.S. Patent No. 8,038,635 to Dellanno teaches a forward head positioning corrective collar including an inflatable assembly. Dellano's inflatable assembly includes multiple inflatable units, each of which can be independently inflated by coupling a tube and bulb to the selected inflatable unit to target a specific vertebra.

Unfortunately, Dellano's collar suffers numerous disadvantages. For example, the pressure of the Dellano collar's inflatable member cannot readily be adjusted by a user while wearing the brace, the inflatable assembly is not suitable for use in any other orthotic device, and each inflatable unit is apparently independently inflated.

Some prior attempts to provide pneumatic solutions to overcome one or more of the disadvantages of traditional orthotic devices are disclosed in U.S. Patent Nos. 3,164,151 to Nicoll, 4,401,111 to Blackstone, 4,732,144 to Cunanan, 6,896,662 to Heffez, and 6,936,002 to Kochamba. Unfortunately, these attempts each suffer from one or more similar disadvantages, including for example, a lack of ability to adjust a positioning of an apex of the inflatable member, and difficulty of use. Document US-A-5 403 266 discloses the most relevant prior art.

Thus, there is still a need for improved braces having a pneumatic element.

### Summary of The Invention

The invention is defined in claim 1.

The disclosure provides apparatus, systems and methods in which an adjustable support for a brace having a pneumatic element includes a pad coupled to at least one of an inflatable member and a pod. The adjustable support could include a front surface and rear surface, wherein the front surface is configured to face a wearer (e.g., contact the wearer's body), and the rear surface faces away from the wearer and is configured to removably couple to a brace panel at one or more positions. The pad of the adjustable support could be oversized with respect to the brace panel such that a slight movement of the adjustable support in vertical, horizontal, diagonal or other directions does not expose the wearer's body to the panel.

The inflatable member can be inflated and deflated using any commercially suitable mechanisms, including for example, a hand pump. The member, when at least partially inflated, can provide an inward / forward force or pressure to a portion of a wearer's body. Where a pod (e.g., a removable or movable pod) is included, the pod can be positioned relative to the inflatable member in any suitable configuration (e.g., between the inflatable member and pad, in front of the inflatable member and pad (i.e., between the inflatable member and the wearer's body), aligned with the inflatable member). The pod can advantageously concentrate, diffuse, move or otherwise direct the inward force applied to the wearer's body.

An adjustable support of the disclosure can be adjustably coupled to one or more panels of various orthotic devices, for example, an inner (or outer) surface of a cervical collar's panel and an inner (or outer) surface of a lumbar support panel. Viewed from another perspective, it is contemplated that an adjustable support can be coupled to and used with two or more different orthotic devices. It is contemplated that an adjustable support of the disclosure could be sized and dimensioned to couple to an inner surface of one or more of a back brace, a lumbar support, a body sock, a knee brace, a leg brace, a wrist wrap, a wrist brace, a cervical brace, a hip brace, a split, an immobilizer, a cast, a foot brace, an ankle brace, or any other suitable orthosis device.

Where the adjustable support is configured to couple to a brace panel at two or more distinct positions, a placement of the central point of the adjustable support, the pad, the pod or the inflatable member relative to the panel could be adjusted. In these and some other contemplated embodiments, it can be advantageous to provide a pad that is oversized relative to the panel along a length, a width, or any other dimension along which the pad can be moved relative to the panel. As the pad will typically be made of a softer material than the panel, the ability to move the pad relative to the panel without exposing the wearer's body to the panel can be essential to a wearer using the adjustable support.

Stated simply, the pad can be advantageously larger than the panel to ensure the panel is sufficiently covered at all times, even when the center portion of the pad is adjusted relative to the panel. Viewed from another perspective, the adjustable member having an oversized pad can be attached to a panel at different positions without causing the panel itself to contact a neck or other portion of the wearer and cause discomfort. This allows a user to attach the adjustable support to a panel in a manner such that a customized force can be applied to a specified portion of the wearer's body without exposing a wearer's skin to the panel's inner surface.

The panel and adjustable support or component thereof (e.g., pad, inflatable member, pod) could include one or more pairs of complementary fasteners to allow the adjustable support to be repositioned relative to the panel in at least one direction. For example, the adjustable support could include a hook or loop fastener on a rear surface, and the panel's inner surface could include a plurality of hook or loop fasteners on a front surface that is complementary to the adjustable support's fastener. As another example, the adjustable support could include a hook or loop fastener on a rear surface, and the panel's front surface could include a larger hook or loop fastener. As yet another example, the adjustable support could include a large hook or loop fastener on a rear surface, and the panel's front surface could include a smaller hook or loop fastener. Viewed from another perspective, the adjustable support's fastener could be smaller than the panel's fastener (e.g., less than 75%, less than 50%, less than 25% of the panel fastener's size), or the panel's fastener could be smaller than the adjustable support's fastener (e.g., less than 75%, less than 50%, less than 25% of the adjustable support's fastener's size).

Various objects, features, aspects and advantages of the inventive subject matter will become more apparent from the following detailed description of preferred embodiments, along with the accompanying drawing figures in which like numerals represent like components.

### Brief Description of the Drawing

**Figure 1** is a perspective view of an adjustable support of the inventive subject matter.
**Figure 2** is a view of a front surface of an adjustable support including a pod and an inflatable member.
**Figures 3A-3D** illustrate some exemplary pods that could be removably inserted into an adjustable support.
**Figure 4** is a side perspective view a collar including an adjustable support of the inventive subject matter.
**Figure 5** is a rear view of the brace of Figure 4.
**Figure 6** is a side view of the brace of Figure 4.

### Detailed Description

The following discussion provides many example embodiments of the inventive subject matter. Although each embodiment represents a single combination of inventive elements, the inventive subject matter is considered to include all possible combinations of the disclosed elements. Thus if one embodiment comprises elements A, B, and C, and a second embodiment comprises elements B and D, then the inventive subject matter is also considered to include other remaining combinations of A, B, C, or D, even if not explicitly disclosed.

Adjustable supports of the inventive subject matter generally include a pad coupled to at least one of (1) an inflatable member that provides an inward force to a part of a body of a wearer, and (2) at least one pod that can be placed relative to the inflatable member to concentrate, diffuse or otherwise direct the inward force provided by the inflatable member. In some preferred embodiments, the adjustable support is configured to removably or adjustably couple with at least one panel of at least one brace such that the adjustable support is held in place at a desired position on a wearer's body.

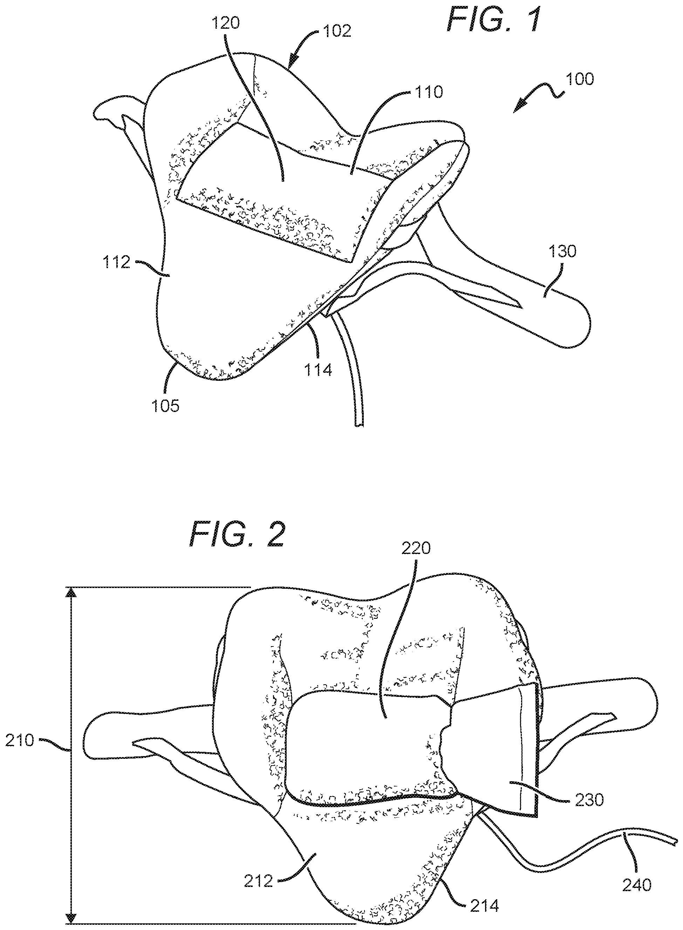

**Figure 1** illustrates a contemplated embodiment of an adjustable support. Adjustable support 100 comprises a rear surface 114, a front surface 112, a pad 102 including a padding material (e.g., foam) and an outer material 105, and an inflatable member 110 coupled to pad 102 (e.g., disposed within the outer material 105). It should be appreciated that the inflatable member could be attached or coupled to pad 102 in any suitable manner. For example, the inflatable member could be disposed in a pocket of pad 102's outer material 105, disposed within pad 102's padding material, or removably attached to pad 102 at one or more location via one or more complementary fasteners on inflatable member 110 and pad 102 (e.g., hook and loop, adhesive, button, snap, sewing, etc.).

Inflatable member 110 has an apex 120, which is the portion or point that has the greatest thickness when inflatable member 110 is fully inflated (e.g., the central point). Viewed from another perspective, an apex is a portion or a point that can provide the greatest pressure or force to a wearer when the adjustable support is worn and the inflatable member 110 is fully inflated. It should be appreciated that an inflatable member could include more than one apex, for example, where the inflatable member comprises two bladder portions coupled via an air passage.

Adjustable support 100 also includes at least a first fastener 130 that is sized and dimensioned to couple with a panel or other portion of at least one orthosis device. Where the adjustable support 100 can be movably coupled to a panel as further described below, it is contemplated that the apex or central point of the inflatable member can be adjustable relative to a central point of the panel. Additionally, where a pod is coupled to the adjustable support 100 (e.g., inserted into a pocket or other compartment of the adjustable support), an apex or central point of the pod can be adjustable relative to the central point of the panel.

As used herein, the term "pod" should be interpreted broadly to include any item that modifies a force applied to a part of a wearer's body by an inflatable member. The pod could be removable, insertable or movable with respect to a pad or inflatable member. In some preferred embodiments, a pod can be contoured to the anatomy of a part of a wearer's body to which it can be applied.

In some embodiments, an adjustable support or pad could include an outer material including a single pocket or a plurality of pockets (e.g., 2 pockets, 3 pockets, 4 pockets, 5 pockets), each of which are sized and dimensioned to receive at least one of an inflatable member and a pod such that they can be repositioned with respect to the pad.

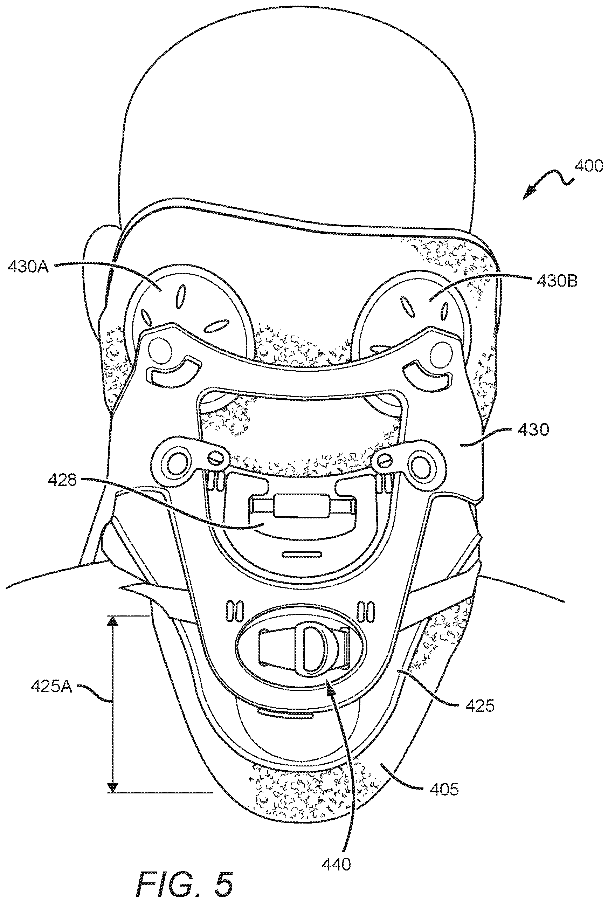

**Figure 2** illustrates an front surface 212 of another embodiment of an adjustable support of the inventive subject matter, which includes a pod (not shown) and an inflatable member 230, each of which are disposed in pocket 220. Rear surface 214 is not shown but is the surface of the adjustable support that would face away from, and not directly contact the neck of the wearer when worn. For example, the rear surface 214 could contact one or more panels such as upper panel 430 and lower panel 425, similarly to what is shown in Figure 5. As another example, the rear surface 214 could comprise a rear surface or outer surface portion of a brace that is not partially or entirely covered by a panel.

The pod can be disposed in a pocket 220 of the pad, and be positioned relative to the inflatable member such that the pod is closer to the wearer's neck than inflatable member 230 when the brace is worn. In some other embodiments, the pod can be disposed further away from the wearer than inflatable member 230, for example within the padding material of the pad, or between the inflatable member and the padding material. The pod can be configured to diffuse, concentrate or otherwise adjust/direct an inward pressure that is applied to the wearer's body via the inflatable member when the brace is worn. Viewed from another perspective, a brace including an inflatable member and a pod can be advantageous over known braces including an inflatable member because, for example, the pod allows a user to have greater control over the pressure applied via the inflatable member.

Where the adjustable support is movably coupled to a panel of an orthosis device such that the panel contacts or at least partially overlies the outer surface of the adjustable support, it is sometimes preferred that a vertical length of the pad 210 (e.g., the longest length of the pad, the midline of the pad, etc.) will be at least 0.5 inch, at least 1 inch, or even 1.5 or more inches greater than a vertical length of the panel (e.g., the longest length of the panel, the midline of the panel, etc.). This advantageously allows some vertical adjustment of the adjustable support relative to the panel (e.g., at least 0.5 inch, at least 1 inch, at least 1.5 inch, etc.) without exposing the wearer's skin to the panel. 1.0 inch corresponds to 2.54 centimetres.

Additionally or alternatively, a horizontal length of the pad 210 (e.g., the longest length of the pad, the midline of the pad, etc.) can be at least 0.5 inch, at least 1 inch, or even 1.5 or more inches greater than a horizontal length of the panel (e.g., the longest length of the panel, the midline of the panel, etc.). This advantageously allows some horizontal adjustment of the adjustable support relative to the panel (e.g., at least 0.5 inch, at least 1 inch, at least 1.5 inch, etc.) without exposing the wearer's skin to the panel. Additionally or alternatively, a diagonal length of the pad 210 (e.g., the longest length of the pad, the midline of the pad, etc.) can be at least 0.5 inch, at least 1 inch, or even 1.5 or more inches greater than a diagonal length of the panel (e.g., the longest length of the panel, the midline of the panel, etc.). This advantageously allows some horizontal adjustment of the adjustable support relative to the panel (e.g., at least 0.5 inch, at least 1 inch, at least 1.5 inch, etc.) without exposing the wearer's skin to the panel.

Viewed from another perspective, it is contemplated that a portion of an outer perimeter of the rear surface or even the entire outer perimeter of the rear surface of the pad could be visible even when a brace panel overlies the pad.

Some contemplated pods include hot, cold or temperature controlled packs that vary in size and shape and can be placed in a pocket of the pad in front of at least a portion of an inflatable member, and activated. Other contemplated pods include, for example, inflatable members of various shapes and sizes, gel-filled or other fluid-filled members of various shapes and sizes, sand, filled members of various shapes and sizes (e.g., filled with beads, pellets, polyurethane foam pieces), hard materials of various shapes and sizes, or high density foam or other materials of various shapes and sizes.

A pod's outer lining, where included, can be made of any suitable material(s), including for example, Nylon™, spandex, Lycra™, or plastic. In some preferred embodiments, a pod is a gel or fluid filled pod including an outer lining material. The distribution of the gel or liquid within the pod could be consistent or could vary within the pod's internal volume, along the length or width of the pod. In some embodiments, the gel or fluid could fill only a portion of the pod's volume (e.g., less than 90%, less than 80%, less than 75%, less than 50%) such that the pod conforms to the shape of the wearer where the brace is worn.

While the examples described herein focus primarily on pods that are insertable and removable from a pocket of an adjustable support, it should be appreciated that the pods can be coupled with a pad in any suitable manner, including via hook and loop or other fasteners. In some embodiments, the pod could be attached to the pad in a fixed position, and a position of the inflatable member could be adjustable.

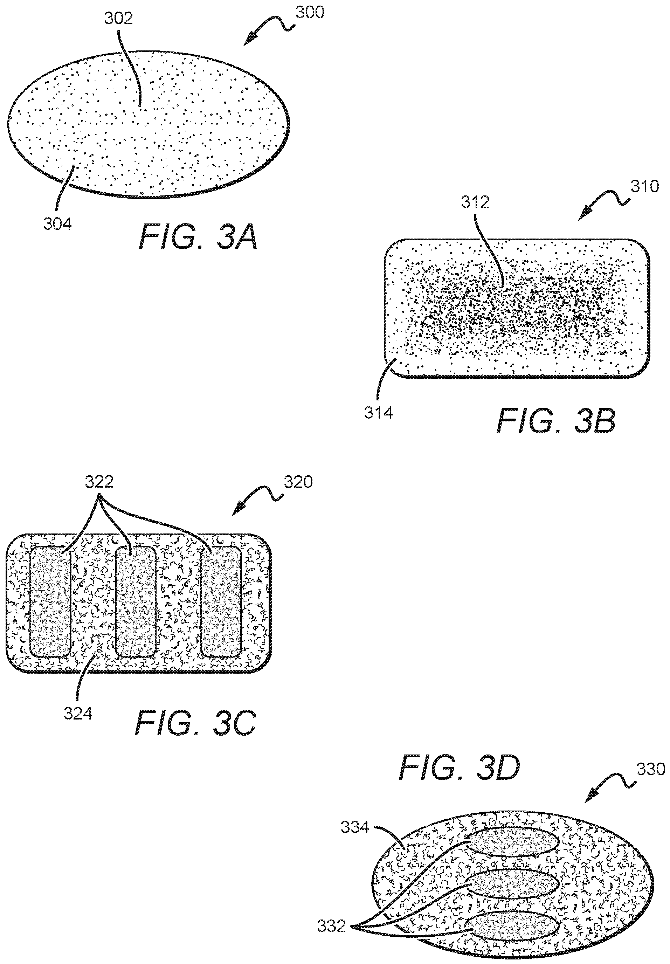

Some exemplary pod shapes are shown in **Figures 3A-3D** having a filler material (e.g., gel, silicon gel, fluid, beads, pellets, sand) distributed within an internal space of a pod in various manners. Contemplated pods can have at least one of (a) fixed tightly packed (filled) and loosely packed portions, (b) a consistent volume of filler material throughout the interior volume of the pod, (c) a consistent volume of filler material throughout a portion of the interior volume of the pod (e.g., at least 25%, at least 50%, at least 75%, at least 80%, at least 90%), (d) an inconsistent volume of filler material throughout the interior volume of the pod, (e) a filler material that can move or flow within the pod such that the pod can conform based on a pressure applied to the pod, and (e) a filler material that substantially does not move or flow within the pod when the pod is worn and a pressure is applied to the pod.

In Figure 3A, pod 300 has a rounded shape and includes a fluid filler material that can move within the volume of pod 300 based on the pressures applied to different portions of pod 300. When the brace is worn by a wearer and greater pressure is applied to the outer perimeter 304, some of the fluid from the outer perimeter 304 can flow to the central area 302 such that the outer perimeter 304 has a reduced thickness and central area 302 has a greater thickness. Where a greater pressure is applied to central area 302, it is contemplated that the fluid from the central area could move to outer perimeter 304. Additionally or alternatively, the fluid could flow from one side of the pod to another or have any suitable distribution based on a pressure that is applied.

Where desirable, a pod could be sectioned via, for example, stitching or adhesive, to keep the filler material contained within predetermined sections of the pod's interior volume. In some embodiments, the tightly packed or filled portion can be separated from a loosely packed or filled portion via a wall (e.g., stitching or adhesive) that extends around the entire tightly packed or filled portion. In some other embodiments, the tightly packed or filled portion can be separated via a wall that extends around only a portion of the tightly packed or filled portion such that some gel, fluid, pellets or beads can move between the portions when enough pressure is applied.

Pod 310 is more rectangular in shape, includes rounded edges, and has a tightly filled (thicker) portion 312 that is sectioned off from less filled (thinner) portion 314. It should be appreciated that a pod could include any ratio of tightly filled and loosely filled portions (e.g., at least 90% tightly filled (at least 90 : 10), at least 80% tightly filled (at least 80 : 20), at least 50% tightly filled (at least 50 : 50), less than 40% tightly filled (less than 40 : 60), less than 20% tightly filled (less than 20 : 80), or less than 10% tightly filled (less than 10 : 90)).

It should also be appreciated that a pod could be any suitable size relative to an inflatable member. For example, the pod could have a width that is smaller than (e.g., less than 90% of, less than 80% of, less than 60% of, less than 50% of, less than 25% of), the same as, or even greater than (e.g., at least 5% greater than, at least 10% greater than, at least 15% greater than, at least 20% greater than, at least 25% greater than, at least 50% greater than) a width of the inflatable member. Where a pod is larger than an inflatable member (e.g., having a greater length or a greater width), it is contemplated that the pod could diffuse a force applied by an inflatable member. Where a pod is smaller than an inflatable member (e.g., having a smaller length or a smaller width), it is contemplated that the pod could concentrate the force applied by an inflatable member.

Pod 320 illustrates a pod filled with beads, pellets, sand or other solid filler material, which includes a plurality of tightly packed portions 322 placed in a horizontal configuration, each tightly packed portion surrounded by a loosely packed portion 324. Pod 330 similarly is filled with a solid filler material, and includes a plurality of tightly packed portions 332 placed in a vertical configuration, each tightly packed portion surrounded by a loosely packed portion 334.

In some aspects of the inventive subject matter, a pod can include a loosely packed portion (or a thinner, softer or even empty portion) that is sized and dimensioned to overlie a bone of a wearer's body. The loosely packed portion can be partially or entirely bordered by tightly packed portions (or fuller, denser, harder or thicker portions) such that the loosely packed or thinner portion overlies the bone and the pressure applied on the bone by the brace is minimized. In other aspects, where it is desirable to apply pressure to a bone, a tightly packed portion could be sized and dimensioned to overlie a bone of a wearer's body, and the tightly packed portion could be partially or entirely bordered by loosely packed portions. It should be appreciated that a single pod (e.g., pod 320) can achieve both of the above via an adjustment of the pod (or even the adjustable support) relative to at least one of the targeted portion of the wearer's body and a central point of a brace panel to which the adjustable support is coupled.

In some embodiments, for example where a pod is larger than an inflatable member, the pod can diffuse an inward force that is applied via the inflatable member when a brace is worn. In some embodiments, for example where a pod has a smaller surface area than an inflatable member or is folded to have a smaller length or width but a greater thickness, the pod can concentrate the inward force applied via the inflatable member when the brace is worn. It should be appreciated that a plurality of varying pods having different dimensions, fillers, materials, firmness, etc. can be insertable or removable from a pocket of some contemplated adjustable supports to allow a user to use different pods to achieve different results. It should also be appreciated that a pod could have a uniform shape, hardness or density, and does not need to include tightly packed and loosely packed portions, thicker and thinner portions, harder and softer portions, or denser and less dense portions. Furthermore, it should be appreciated that a single pod could be used in different configurations (e.g., folded, unfolded) or at different positions to direct a force to be applied by an inflatable member in different ways.

Additionally or alternatively to having tightly filled and loosely filled portions, a pod including a filler material could comprise thicker and thinner portions relative to one another, or even portions with different types of filler materials. In general, the thicker portions could increase a force that is applied to a wearer via the adjustable support, and the thinner portions could lessen the force that is applied to the wearer via the adjustable support. However, it should be appreciated that different portions of a pod could direct a force applied by an inflatable member in various ways (e.g., concentrate, diffuse, increase, decrease) depending on one or more factors such as the shape of the pad, and the position of the pod relative to the inflatable member or pad.

Where a pod does not include filler materials, the pod could nevertheless have a varying density, a varying hardness or a varying thickness across a length of the pod. The pod can comprise a single piece that is made from one or more materials having the same or different qualities (e.g., hardness, density, compressibility). For example, a pod could comprise a combination of a soft foam and a hard foam (relative to the hard foam) that is coupled together and enclosed in a material such as Lycra. The soft foam could direct a force similarly to loosely filled or thinner portions of a filler material pod, while the hard foam could direct a force similarly to tightly filled portions or thicker portions of a filler material pod.

In some aspects of the inventive subject matter, an adjustable support of the inventive subject matter could be used to provide a collar or other brace having a pneumatic element. As one example, a collar can include (1) a panel having a vertical length and outer and inner surfaces - the inner surface facing the wearer when the brace is worn, and (2) a pad adjustably coupled to the panel via one or more fasteners. The pad can include or be coupled to an inflatable member in a manner that inflation of the inflatable member can increase an inward force applied to a neck of a user when the collar is worn.

The pad can advantageously have a vertical length that is at least 0.5 inch, at least 1 inch, or even 1.5 inches or more greater than the vertical length of the panel such that an adjustment of the pad relative to the panel in a vertical direction adjusts an apex of the inflatable member relative to the panel without exposing the user's neck to the inner surface of the panel. Additionally or alternatively, the pad can have a horizontal length that is at least 0.5 inch, at least 1 inch, or even 1.5 inches or more greater than the horizontal length of the panel such that an adjustment of the pad relative to the panel in a horizontal direction adjusts an apex of the inflatable member relative to the panel without contacting the user's neck with the inner surface of the panel.

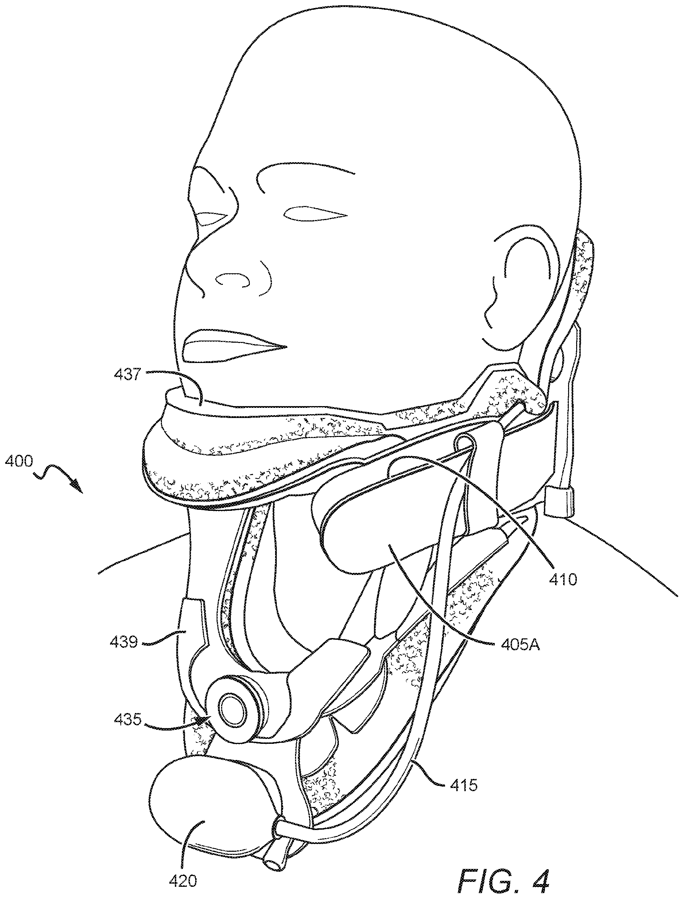

**Figure 4** illustrates an embodiment of a brace including an adjustable support of the inventive subject matter. Brace 400 is a collar having a pneumatic element that includes an adjustable support 405 similar to the adjustable support of Figure 2. The adjustable support includes first fastener 405A, which can thread through an opening or slot included on or coupled to a panel of brace 400 and fasten with second fastener 410 of the same or different panel of brace 400. Preferably, complementary fasteners are included on another portion of the adjustable support and brace 400 such that the adjustable support can be secured to at least two sides, for example left and right sides, of the brace 400. In some contemplated embodiments, the adjustable support 405 can be used to couple the brace's rear panel with the brace's front panel. For example, the adjustable support could include a pad that removably attaches to the rear panel's inner or front surface (e.g., at least one of upper panel 430 and lower panel 425), and a first fastener that threads through an opening of the rear panel and attaches to a fastener of the front panel's outer or rear surface. As another example, the adjustable support could include a pad that removably attaches to the rear panel's inner surface (e.g., at least one of upper panel 430 and lower panel 425), and a first fastener that threads through an opening of a front panel and attaches to a fastener of the rear panel's outer surface.

Tube 415 and pump 420 can be used by the wearer to inflate or deflate an inflatable member of adjustable support 405 to customize an inward pressure applied to the wearer's neck. The tube and pump can be attached to the inflatable member or can be removably coupled thereto. Additionally or alternatively, a housing or holder can be included on the brace, for example as part of a panel, to house or hold the pump when not in use. Brace 400 further includes a front panel portion having first adjustment mechanism 435 that allows a user to adjust a vertical position of a chin piece 437 relative to a collar body 439, which are coupled together via one or more pivot points or chin supports. An exemplary adjustment mechanism is a rack and pinion adjustment mechanism found in co-owned U.S. Patent No. 7,674,234, wherein rotation of a pinion causes a rack to move a chin support member, which raises and lowers a chin piece. Other contemplated adjustment mechanisms include those described in U.S. Patent Application Serial Numbers 14/638366, 14/638519, and 14/846609.

**Figure 5** is a rear view of brace 400 as worn by a wearer. The rear portion of brace 400 includes a lower panel 425 coupled to upper panel 430 via second adjustment mechanism 440. First adjustable support 405 is removably attached to lower panel 425's inner surface via one or more suitable fasteners (e.g., hook and loop fasteners, snaps, etc.) and has a vertical length that is greater than the first vertical length 425A of the lower panel 425. The oversized nature of the adjustable support relative to the panel can allow a user to adjust an apex of the adjustable support's inflatable member relative to the panel without contacting the wearer's skin with the first panel's inner surface.

Adjustment mechanism 440 allows a user to adjust a vertical position of the upper panel 430 relative to the lower panel 425. Where an upper panel includes or is coupled with one or more occipital lobe supports (e.g., 430A, 430B as shown in Figure 5), the adjustment mechanism 440 can further allow a user to adjust a vertical position of each of the occipital lobe supports 430A, 430B relative to the lower panel 425 with a single vertical adjustment. Brace 400 also includes a junction support 428 which can be configured to couple with a front or real portion of the brace. In the embodiment shown, junction support 428 couples to the rear portion of brace 400 and can comprise or couple with any suitable accessory, including for example, a belt, a back support, a strut, a chest supporting structure, or a realigning member.

It should be appreciated that any suitable adjustment mechanism 440 could be provided in a brace of the inventive subject matter, including the protrusion-notch-locking member adjustment mechanism shown in Figure 5. Upper panel 430 includes a slidable locking member that includes or is otherwise coupled with a protrusion (not shown) sized and dimensioned to fit at least partially within some or all notches (not shown) of lower panel 425. Preferably, the protrusion is located on or against the inner surface of upper panel 430, the notches are located on an outer surface of lower panel 425 in a vertical orientation, and the locking member locks the protrusion at least partially within one of the notches. An exemplary rear portion and adjustment mechanism can be found in Applicant's co-owned U.S. Patent Application Publication No. 2013/0261519.

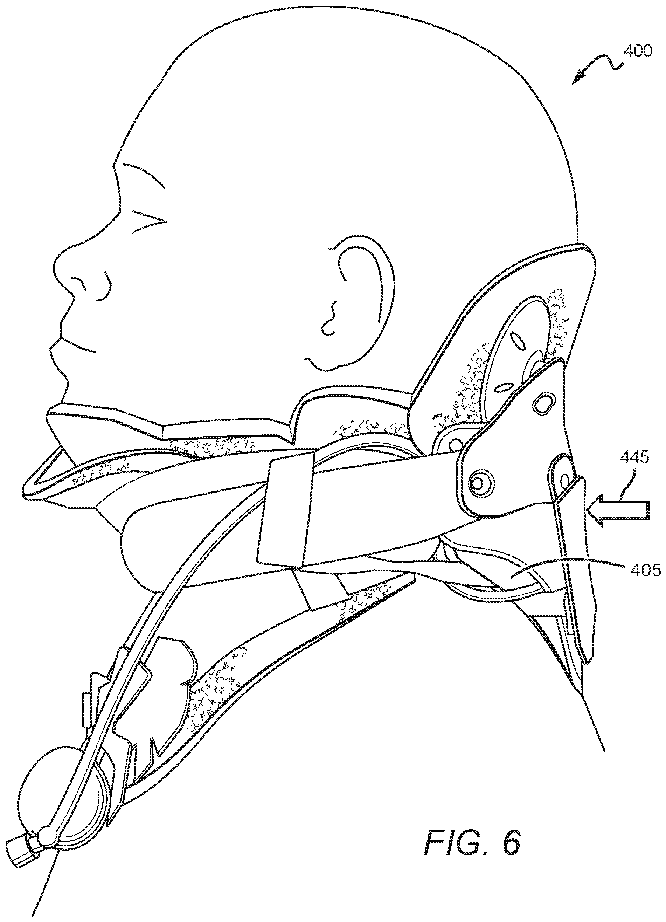

**Figure 6** is a side view of brace 400, which illustrates a force that can be applied to a wearer's body via the adjustable support 405. Where first adjustable support 405 is removably attached to an inner surface of a brace panel (as shown in Figure 6 with respect to lower panel 425), inflation of the adjustable support's inflatable member will cause an inward force 445 to be applied to a portion of the wearer's body over which the inflatable member is placed. Where a pod is included to be positioned in front of at least a portion of an inflatable member (closer to the wearer), the pod can diffuse, concentrate, or otherwise direct the inward force 445 that is applied to the portion of the wearer's body based at least in part on the pod's size, shape, position, and the distribution of air or other filler within the pod. It is also contemplated that one or more pods could be included to be positioned under an inflatable member (closer to the wearer), over an inflatable member (away from the wearer), in line with an inflatable member (at least in part), or combinations thereof.

It should be appreciated that while examples herein are generally directed towards collars with a pneumatic element configured to apply an inward force to a neck of a wearer, an adjustable support of the inventive subject matter could be utilized with any suitable brace to provide adjustable support and force or pressure to any desired portion of a wearer's body. As some non-limiting examples, an adjustable support of the inventive subject matter could be sized and dimensioned to couple with one or more of a scoliosis brace, a cervical collar, a lumbar support brace, a knee brace, an ankle brace or wrap, a torso brace and a torso support. Viewed from another perspective, the adjustable support could be coupled to two or more braces and include an inflatable member to provide a force or pressure to one, some or all of a back, a neck, a lower back, a knee, an ankle, and a torso of a wearer. The adjustable support could couple with the braces via any suitable fasteners or attachment mechanisms, including for example, hook and loop fasteners, snaps, buttons, threading, sewing, glue, adhesives, or any other suitable means.

## Claims

1. A collar for a wearer, comprising: a pad; a pod;
a panel having an outer surface and an inner surface;
an inflatable member (230) configured to couple to the pad and the panel, and apply an inward force to the wearer;
wherein a position of the inflatable member relative to the panel is adjustable;
wherein the inflatable member is further coupled to the pod that directs the inward force that is applied to the neck of the user, and wherein
the pad comprises a pocket (220) that is sized and dimensioned to receive the pod.

2. The collar of claim 1, wherein the inflatable member comprises a pump that is inflatable by the user wearing the collar, and/or wherein a position of the inflatable member relative to the panel is adjustable by at least 1.27 cm (0.5 inch).

3. The collar of claim 1 or claim 2, wherein the pod is sized and dimensioned to cooperate with the inflatable member to diffuse the inward force that is applied to the neck of the user.

4. The collar of claim 1 or claim 2, wherein the pod is sized and dimensioned to cooperate with the inflatable member to concentrate the inward force that is applied to the neck of the user.

5. The collar of any one of claims 1-4, wherein the inflatable member is positioned between the pod and the panel.

6. The collar of claim 1, wherein the panel comprises a first length, wherein the pad comprises a second length, wherein the first length of the panel overlies the second length of the pod, and wherein the second length is at least 1.27 cm (0.5 inch) greater than the first length.

7. The collar of claim 1, wherein the pad has a surface area greater than a surface area of the panel.

8. The collar of claim 1, wherein the pod comprises at least one of a gel and a fluid.

## Patentansprüche

1. Manschette für einen Träger, die Folgendes beinhaltet:
ein Pad;
ein Pod;
ein Panel, das eine Außenoberfläche und eine Innenoberfläche aufweist;
ein aufblasbares Glied (230), das konfiguriert ist, um mit dem Pad und dem Panel gekoppelt zu werden und eine Einwärtskraft auf den Träger auszuüben;
wobei eine Position des aufblasbaren Glieds relativ zu dem Panel verstellbar ist;
wobei das aufblasbare Glied ferner mit dem Pod gekoppelt ist, das die Einwärtskraft, die auf den Nacken des Benutzers ausgeübt wird, lenkt, und wobei das Pad eine Tasche (220) beinhaltet, die bemessen und dimensioniert ist, um das Pod aufzunehmen.

2. Manschette gemäß Anspruch 1, wobei das aufblasbare Glied eine Pumpe beinhaltet, die von dem Benutzer, der die Manschette trägt, aufblasbar ist, und/oder wobei eine Position des aufblasbaren Glieds relativ zu dem Panel um mindestens 1,27 cm (0,5 Zoll) verstellbar ist.

3. Manschette gemäß Anspruch 1 oder Anspruch 2, wobei das Pod bemessen und dimensioniert ist, um mit dem aufblasbaren Glied zusammenzuwirken, um die Einwärtskraft, die auf den Nacken des Benutzers ausgeübt wird, zu streuen.

4. Manschette gemäß Anspruch 1 oder Anspruch 2, wobei das Pod bemessen und dimensioniert ist, um mit dem aufblasbaren Glied zusammenzuwirken, um die Einwärtskraft, die auf den Nacken des Benutzers ausgeübt wird, zu konzentrieren.

5. Manschette gemäß einem der Ansprüche 1-4, wobei das aufblasbare Glied zwischen dem Pod und dem Panel positioniert ist.

6. Manschette gemäß Anspruch 1, wobei das Panel eine erste Länge beinhaltet, wobei das Pad eine zweite Länge beinhaltet, wobei die erste Länge des Panels über der zweiten Länge des Pods liegt und wobei die zweite Länge mindestens 1,27 cm (0,5 Zoll) größer als die erste Länge ist.

7. Manschette gemäß Anspruch 1, wobei das Pad einen Oberflächenbereich aufweist, der größer als ein Oberflächenbereich des Panels ist.

8. Manschette gemäß Anspruch 1, wobei das Pod mindestens eines von einem Gel und einem Fluid beinhaltet.

## Revendications

1. Collier pour un porteur, comprenant :
un tampon ;
une nacelle ;
un panneau possédant une surface extérieure et une surface intérieure ;
un élément gonflable (230) configuré pour se coupler au tampon et au panneau, et appliquer une force vers l'intérieur contre le porteur ;
une position de l'élément gonflable relativement au panneau étant ajustable ;
l'élément gonflable étant couplé en outre à la nacelle dirigeant la force intérieure appliquée sur le col de l'utilisateur, et le tampon comprenant une poche (220) dimensionnée pour recevoir la nacelle.

2. Collier selon la revendication 1, l'élément gonflable comprenant une pompe gonflable par l'utilisateur portant le collier, et/ou une position de l'élément gonflable relativement au panneau pouvant être ajustée d'au moins 1,27 cm (0.5 pouce).

3. Collier selon la revendication 1 ou la revendication 2, la nacelle étant dimensionnée pour coopérer avec l'élément gonflable de façon à diffuser la force vers l'intérieur appliquée sur le col de l'utilisateur.

4. Collier selon la revendication 1 ou la revendication 2, la nacelle étant dimensionnée pour coopérer avec l'élément gonflable afin de concentrer la force vers l'intérieur appliquée sur le col de l'utilisateur.

5. Collier selon une quelconque des revendications 1-4, l'élément gonflable étant positionné entre la nacelle et le panneau.

6. Collier selon la revendication 1, le panneau comprenant une première longueur, le tampon comprenant une deuxième longueur, la première longueur du panneau recouvrant la deuxième longueur de la nacelle, et la deuxième longueur dépassant la première longueur d'au moins 1,27 cm (0,5 pouce).

7. Collier selon la revendication 1, la superficie du tampon étant supérieure à une superficie du panneau.

8. Collier selon la revendication 1, la nacelle comprend au moins un d'un gel et d'un fluide.
